Europäisches Patentamt

European Patent Office

Office européen des brevets

(11) Publication number: **0 140 486**
**B1**

(12) **EUROPEAN PATENT SPECIFICATION**

(45) Date of publication of patent specification: **20.09.89**

(21) Application number: **84305209.3**

(22) Date of filing: **31.07.84**

(51) Int. Cl.⁴: **A 61 K 6/00, C 09 J 3/04, C 09 J 3/16**

(54) Denture adhesive composition.

(30) Priority: **01.08.83 US 519331**

(43) Date of publication of application:
**08.05.85 Bulletin 85/19**

(45) Publication of the grant of the patent:
**20.09.89 Bulletin 89/38**

(84) Designated Contracting States:
**BE CH DE FR GB IT LI NL SE**

(56) References cited:
**DE-A-3 004 161**
**US-A-2 978 812**
**US-A-3 440 065**
**US-A-4 304 902**

(73) Proprietor: **HERCULES INCORPORATED**
**Hercules Plaza**
**Wilmington Delaware 19894 (US)**

(72) Inventor: **Desmarais, Armand Joseph**
**3419 Pebble Beach Drive Fairway Falls**
**Wilmington Delaware 19808 (US)**

(74) Representative: **De Minvielle-Devaux, Ian Benedict Peter et al**
**CARPMAELS & RANSFORD 43, Bloomsbury Square**
**London WC1A 2RA (GB)**

Courier Press, Leamington Spa, England.

EP 0 140 486 B1

**Description**

This invention relates to denture adhesive compositions in powder and cream form having excellent adhesion properties and improved mouthfeel.

The use of dental prostheses as replacements for teeth is now widespread. Advances in the art, particularly in plastic and alloy chemistry, have made it possible to produce dentures which not only function better, but are markedly improved in appearance. The most common types of dental prostheses are (1) bridgework, fixed or removable, which are generally used to replace up to three missing teeth; (2) partial dentures, which are removable and are used when several teeth are missing; and (3) full dentures which are removable when all teeth of the upper or lower jaw or both have been removed or otherwise lost.

Concomitantly with the use of dentures, especially full dentures, denture adhesive compositions, first in powder form and later in cream form, were developed. Although there has been some dissent in the dental community as to whether denture adhesives should be used, they have come to be used extensively by the dental plate wearers. Basically, the dissenting opinion of the dental authorities rests on the belief that such adjuvant compositions are used as or become a substitute for proper fitting dentures or plates, and that the prior art denture adhesive compositions do not perform well under the wide range of conditions present in the oral cavity over any appreciable period of time, i.e., at least eight hours or more.

The denture adhesive is used by applying it to the face of the denture or plate which is particularly adapted to contact and mold itself to the contour of a particular oral surface in the mouth, and then placing the denture in the mouth against and in contact with the oral surface.

Desirably, a denture adhesive should not be readily soluble in the fluids present in or taken into the mouth, should be resistant to the extreme changes in the temperature of the fluids taken in the mouth, and should be able to accommodate variations in the denture wearer's diet which results in diverse chemical characteristics, including pH. Further, the denture adhesive should have good adhesion or cohesion properties over a long period of time. These performance criteria are essential if the denture or plate is to be held in its place in the mouth by the denture adhesive composition, particularly during the mastication of foods and the drinking of beverages.

For years the majority of denture adherent powders or creams used to secure dentures in the mouth were prepared from finely ground particles of natural gums, such as karaya, acacia, guar and tragacanth. The finely ground particles were dispersed in an anhydrous cream base, usually petrolatum, when the cream form was desired.

More recently, denture powders and creams have been prepared with cellulosic materials, such as sodium carboxymethyl cellulose, hydroxyethylcellulose, and hydroxypropylcellulose either alone or in combination with ethylene oxide homopolymers, acrylamide homopolymers and copolymers, or maleic anhydride derivatives to improve the adhesion properties of the denture powders and creams. Generally, such materials are dispersed in petrolatum, in a mineral oil, or in a mixture of petrolatum with a mineral oil as the carrier. Optionally, the mineral oil can be thickened with polyethylene.

These denture adhesive cream formulations provided some improvement over the traditional compositions containing only a natural gum in the powder form or a natural gum in a petrolatum carrier in the case of the cream form. However, these compositions only effectively secure the dentures in the mouth over short periods of time. Therefore, it has generally been necessary to apply more than one application of the denture adhesive per day in order to obtain and maintain sufficient adhesion throughout the day. Multiple applications of the adhesive are not only inconvenient, but are usually impractical if not impossible. Moreover, many of the commercially available denture adhesives have a greasy or oily mouthfeel. Hence, there is a need for a denture adhesive which exhibits superior adhesive properties over long periods of time, and which does not feel oily or greasy to the mouth tissues.

Moreover, it was and still is essential that the natural or synthetic polymer used in denture adhesive powders and creams be ground sufficiently fine to avoid a gritty mouthfeel. Generally, the particle size distribution is such that the polymeric material will pass through a 100 mesh to 200 mesh screen.

The present invention provides a denture adhesive composition containing from 35% to 85% of a petrolatum, and 0% to 15% of a material selected from the group consisting of mineral oil and mineral oil admixed with a polyethylene, characterized in that it also contains from 5% to 50% of a hydrophobically modified water-soluble polymer selected from the group consisting of hydrophobically modified cellulose ether, as defined hereinafter, and a copolymer of ethylene oxide and at least one long chain 1,2-epoxyalkane, as defined hereinafter.

The present invention also provides a dry denture adhesive composition characterized in that it contains as its principal ingredient a hydrophobically modified water-soluble polymer selected from the group consisting of a hydrophobically modified cellulose ether, as defined hereinafter, and a copolymer of ethylene oxide and at least one long chain 1,2-epoxyalkane, as defined hereinafter, the composition also containing from 10% to 95% by weight of an alkali metal or calcium salt of carboxymethyl cellulose and/or 0.1% to 60% by weight of a filler.

The compositions of this invention afford superior adhesion or cohesion properties under the variable environmental conditions encountered in the mouth over a substantially increased period of time. Moreover, the compositions have a substantially reduced greasy or oily mouthfeel.

Additionally, the hydrophobically modified water-soluble polymers used in the denture adhesive compositions of this invention are not as hard a particulate matter as the conventional materials. Hence, they do not require as fine a grind as the conventional materials. For example, particles of the hydrophobically modified water-soluble polymers that pass through an 80 mesh screen, but are retained on a 100 mesh screen are suitable for this use.

The hydrophobically modified cellulose ethers — which include water-soluble alkyl and hydroxyalkyl celluloses, such as methyl, hydroxyethyl, and hydroxypropyl cellulose — are substitued with a hydrocarbon radical having from 8 to 25 carbon atoms, preferably from 12 to 20 carbon atoms, in an amount from 0.2 weight percent to the amount which renders the cellulose ether less than 1% by weight soluble in water. The cellulose ether to be modified is typically of low to medium molecular weight, i.e., less than about 800,000, preferably 20,000 to about 500,000. The preferred cellulose ether substrate is hydroxyethyl cellulose.

The term "hydrocarbon radical" as used herein is meant to include the hydrocarbon portion as well as any other moiety present, such as an ester, ether or urethane moiety, as a result of the particular compound used to further substitute the cellulose ethers.

The hydrophobically modified cellulose ethers can be prepared by the method set forth in U.S. Patent 4,228,277.

The ethylene oxide copolymers are the copolymers of ethylene oxide with at least one 1,2-epoxyalkane containing from 8 to 25 carbon atoms, preferably from 12 to 20 carbon atoms, which copolymers contain, by weight, from 96% to 99.9% ethylene oxide and from 4% to 0.1% of the 1,2-epoxyalkane. Preferably they have a molecular weight from about 20,000 to about 1,000,000.

The ethylene oxide copolymers can be prepared by the method set forth in U.S. Patent 4,304,902.

The hydrophobically modified water-soluble polymer can be combined with a finely divided alkali metal (preferably sodium) salt or calcium salt of carboxymethyl cellulose. Preferably the salt of carboxymethyl cellulose has a degree of substitution (D.S.) of at least 0.3, a molecular weight of from about 50,000 to about 1,250,000, and is present in an amount from about 10% to about 45% when used in the cream form and from about 10% to about 95% when used in the dry form. Most preferably the D.S. is from about 0.6 to about 1.6 and the molecular weight is from about 300,000 to about 1,250,000. Degree of substitution is the number of carboxymethyl groups per anhydroglucose unit of the cellulose molecule.

The salt of carboxymethyl cellulose can be prepared by the method described in R. L. Whistler & J. N. BeMiller "Industrial Gums", 696 (2d ed. 1973).

The polymers used in the composition of this invention may be used directly as a denture adhesive in powder form. Alternatively, they can be mixed with an anhydrous cream carrier or vehicle to prepare a denture adhesive in cream form.

The carrier or vehicle can be petrolatum or petrolatum combined with mineral oil. The mineral oil can be admixed with a polyethylene. Generally, the carrier is present at a concentration of from 35% to 85% by weight. When a combination of petrolatum and mineral oil, or petrolatum and mineral oil containing a polyethylene, is used as the carrier, the petrolatum is preferably present at a concentration of from 40% to 80% and the mineral oil or mineral oil containing a polyethylene at a concentration of from 1% to 15%, for example up to 10%, by weight.

In addition, the denture adhesive of this invention can contain other water swellable or soluble polymers, such as polyoxyethylene, polyacrylamide, acrylamide-acrylic acid copolymers, and maleic anhydride derivatives. Other excipient materials, such as fillers, flavoring agents, coloring agents and preservatives can also be included in the compositions of this invention. Typical fillers include dicalcium phosphate, calcium carbonate, and talc. The fillers can be present in an amount from 0.1% to 60% in dry denture adhesives, and from 0.1% to 20% in cream denture adhesives.

To further illustrate this invention, various illustrative examples are set forth below.

All parts and percentages are by weight, unless otherwise specified, throughout the specification.

### Example 1

This example illustrates an embodiment of the denture adhesive composition of this invention and how to prepare it.

Thirty-five (35)% hydrophobically modified hydroxyethyl cellulose having a molecular weight of 200,000 and having 0.7% by weight of $C_{16}$ alkane is added to 65% petrolatum in a mixing kettle and stirred until the ingredients are thoroughly mixed.

### Example 2

This example illustrates another embodiment of the denture adhesive composition of this invention.

The composition is prepared according to the procedure and formulation of Example 1 with the exception that a hydrophobically modified ethylene oxide polymer having a molecular weight of 50,000 and having 0.7% by weight of a 1,2-epoxyalkane is used instead of the modified hydroxyethyl cellulose of Example 1.

### Example 3

This example illustrates another embodiment of the denture adhesive composition of this invention.

The composition is prepared according to the procedure of Example 1 with the formulation of Example 1 except that 10% of the hydrophobically modified hydroxyethyl cellulose and 50% petrolatum is used, and 40% of the sodium salt of carboxymethyl cellulose (CMC) having a D.S. of 0.7 is added.

### Example 4

This example illustrates another embodiment of the denture adhesive composition of this invention.

The composition is prepared according to the procedure of Example 1 with the formulation of Example 3 except that 10% of the hydrophobically modified ethylene oxide polymer of Example 2 is used instead of the hydrophobically modified hydroxyethyl cellulose.

### Example 5

This example illustrates another embodiment of the denture adhesive composition of this invention.

The composition is prepared by dry blending 20% of the hydrophobically modified hydroxyethyl cellulose of Exmaple 1, 50% CMC having a D.S. of 0.7, and 30% dicalcium phosphate.

### Example 6

This example illustrates another embodiment of the denture adhesive composition of this invention.

The composition is prepared according to the procedure of Example 5 with the formulation of Example 5 except that the hydrophobically modified ethylene oxide polymer of Example 2 is used instead of the hydrophobically modified hydroxyethyl cellulose.

To characterize the compositions of this invention the following tests were conducted.

Adhesive test: A 3″ × ¾″ (7.62 cm × 1.92 cm) methylmethacrylate plate and a 3″ × ¾″ (7.62 cm × 1.91 cm) spunbonded polyester sheet are dipped in distilled water. A 0.5 g. sample of the denture adhesive composition is placed on and spread over a 2″ (5.08 cm) length of the plate. The plate is covered with the spunbonded polyester sheet, and then manually pressed flat.

A test plate is then placed in a crystallization dish filled halfway with distilled water and equipped with a magnetic stirrer. The dish is covered with aluminium foil and placed on a stirrer base which is set for light agitation. Stirring is maintained for 4 hours to age the samples.

After 4 hours, the test plates are removed from the dish and blotted dry with paper towels. The bond strength (adhesion) is then measured on an Instron tester, equipped with Microcon 2 data acquisition system, at a rate of pull of 5″ (12.7 cm)/minute. The peak load is printed out in grams of force.

Tack and stiffness test: One gram (1.0 g.) SD Alcohol 40 is added to 10 g. of the denture adhesive composition in a 150 ml. beaker and stirred with an aluminum spatula until the ingredients are thoroughly mixed. Nineteen grams of distilled water (19.0 g.) are added and stirring is continued until a homogeneous mixture is obtained. The mixture is then transferred to a Petri dish and evenly distributed over the bottom of the Petri dish with the spatula. The dish is covered and allow to set for 24 hours.

The dish is uncovered and centered on the sample plate of a Voland Stevens LFRA texture analyzer. A ½″ (1.27 cm) diameter butyl methacrylate probe is activated at 1 mm/sec. and placed at a depth of 4 mm into the sample. The resistance and adhesion to the probe, which is a measurement of stiffness and tack, respectively, is recorded in grams.

The test results for the compositions of this invention embodied in Examples 1—6 are set forth in Table 1 below.

|  |  |  |  |
|---|---|---|---|
| Table 1 | | | |
| Example No. | Adhesion (g.) | Stiffness (g.) | Tack (g.) |
| 1 | 95 | 210 | 35 |
| 2 | 110 | 180 | 50 |
| 3 | 140 | 280 | 45 |
| 4 | 160 | 230 | 55 |
| 5 | 140 | 290 | 40 |
| 6 | 170 | 210 | 60 |

Thus, this invention provides a novel denture adhesive composition having superior adhesion characteristics.

Features, advantages and other specific embodiments of this invention will become readily apparent to those exercising ordinary skill in the art after reading the foregoing disclosures.

**Claims**

1. A denture adhesive composition containing from 35% to 85% by weight of a petrolatum, and 0% to 15% by weight of a material selected from the group consisting of mineral oil and mineral oil admixed with a polyethylene, characterized in that it also contains from 5% to 50% by weight of a hydrophobically modified water-soluble polymer selected from the group consisting of hydrophobically modified cellulose ether substituted with a hydrocarbon radical having 8 to 25 carbon atoms in an amount from 0.2 weight percent to about the amount which renders the cellulose ether less than 1% by weight soluble in water, and a copolymer of ethylene oxide and at least one long chain 1,2-epoxyalkane having 8 to 25 carbon atoms, and which contains, by weight, from 96% to 99.9% ethylene oxide and from 4% to 0.1% of the 1,2-epoxyalkane.

2. A composition according to claim 1 containing from 40% to 80% by weight of the petrolatum, 0% to 10% by weight of the material selected from the group consisting of mineral oil and a mineral oil admixed with a polyethylene, and from 5% to 35% by weight of the hydrophobically modified water-soluble polymer, characterised in that it also contains from 10% to 45% by weight of an alkali metal or calcium salt of carboxymethyl cellulose.

3. A composition according to claim 2, characterised in that the said salt of carboxymethyl cellulose has a D.S. of at least 0.3.

4. A dry denture adhesive composition characterised in that it contains as its principal ingredient a hydrophobically modified water-soluble polymer selected from the group consisting of a hydrophobically modified cellulose ether substituted with a hydrocarbon radical having 8 to 25 carbon atoms in an amount from 0.2 weight percent to the amount which renders the cellulose ether less than 1% by weight soluble in water, and a copolymer of ethylene oxide and at least one long chain 1,2-epoxyalkane having 8 to 25 carbon atoms, and which contains, by weight, from 96% to 99.9% ethylene oxide and from 4% to 0.1% of the 1,2-epoxyalkane, the composition also containing from 10% to 95% by weight of an alkali metal or calcium salt of carboxymethyl cellulose.

5. A dry denture adhesive composition characterised in that it contains as its principal ingredient a hydrophobically modified water-soluble polymer selected from the group consisting of a hydrophobically modified cellulose ether substituted with a hydrocarbon radical having 8 to 25 carbon atoms in an amount from 0.2 weight percent to the amount which renders the cellulose ether less than 1% by weight soluble in water, and a copolymer of ethylene oxide and at least one long chain 1,2-epoxyalkane having 8 to 25 carbon atoms, and which contains by weight, from 96% to 99.9% ethylene oxide and from 4% to 0.1% of the 1,2-epoxyalkane, the composition also containing 0.1% to 60% by weight of a filler.

6. A composition according to claim 5, characterised in that the filler is dicalcium phosphate, calcium carbonate or talc.

7. A composition according to claim 5 or 6, characterised in that it also contains from 10% to 95% by weight of an alkali metal or calcium salt of carboxymethyl cellulose.

8. A composition according to claim 4 or 7, characterised in that the said salt of carboxymethyl cellulose has a D.S. of at least 0.3.

9. A composition according to claim 2, 3, 4, 7 or 8, characterised in that the salt of carboxymethyl cellulose is a sodium salt.

10. A composition according to any one of claims 1 to 9, characterised in that it contains only the hydrophobically modified cellulose ether as the said water-soluble polymer.

11. A composition according to any one of claims 1 to 10, characterised in that the cellulose ether that is hydrophobically modified is an alkyl or hydroxyalkyl cellulose.

12. A composition according to claim 11, characterised in that the cellulose ether that is hydrophobically modified is hydroxyethyl cellulose.

13. A composition according to any one of claims 1 to 9, characterised in that it contains only the said copolymer of ethylene oxide and 1,2-epoxyalkane as the said water-soluble polymer.

14. A composition according to any one of claims 1 to 13, characterised in that said hydrocarbon radical or 1,2-epoxyalkane has 12 to 20 carbon atoms.


## Patentansprüche

1. Haftzusammensetzung für Zahnprothesen enthaltend von 35% bis 85 Gew.-% eines Petrolatums und 0% bis 15 Gew.-% eines Materials, ausgewählt aus der Gruppe bestehend aus Mineralöl und Mineralöl gemischt mit einem Polyethylen, dadurch gekennzeichnet, daß sie auch enthält von 5% bis 50 Gew.-% eines hydrophob modifizierten wasserlöslichen Polymers ausgewählt aus der Gruppe bestehend aus hydrophob modifizertem Celluloseether, substituiert mit einem Kohlenwasserstoffrest mit 8 bis 25 Kohlenstoffatomen, in einer Menge von 0,2 Gew.-% bis etwa der Menge, die den Celluloseether weniger als 1 Gew.-% löslich in Wasser macht, und einem Copolymer von Ethylenoxid und mindestens einem langkettigen, 1,2-Epoxyalkan mit 8 bis 25 Kohlenstoffatomen, und, das enthält, bezogen auf Gewicht, von 96% bis 99,9% Ethylenoxid und von 4% bis 0,1% des 1,2-Epoxyalkans.

2. Zusammensetzung nach Anspruch 1, enthaltend von 40% bis 80 Gew.p% des Petrolatums, 0% bis 10 Gew.-% des Materials, ausgewählt aus der Gruppe, bestehend aus Mineralöl und einem Mineralöl gemischt mit einem Polyethylen, und von 5% bis 35 Gew.-% des hydrophob modifizierten wasserlöslichen Polymers, dadurch gekennzeichnet, daß sie auch von 10% bis 45 Gew.-% eines Alkalimetall- oder Calciumsalzes von Carboxymethylcellulose enthält.

3. Zusammensetzung nach Anspruch 2, dadurch gekennzeichnet, daß das Salz von Carboxymethylcellulose einen D.S. von mindestens 0,3 hat.

4. Trockene Haftzusammensetzung für Zahnprothesen, dadurch gekennzeichnet, daß sie als ihren Hauptbestandteil enthält ein hydrophob modifiziertes wasserlösliches Polymer, ausgewählt aus der Gruppe, bestehend aus einem hydrophob modifizierten Celluloseether, substituiert mit einem Kohlenwasserstoffrest mit 8 bis 25 Kohlenstoffatomen in einer Menge von 0,2 Gew.-% bis zu der Menge, die den Celluloseether weniger als 1 Gew.-% löslich in Wasser macht, und einem Copolymer von Ethylenoxid und mindestens einem langkettigen 1,2-Epoxyalkan mit 8 bis 25 Kohlenstoffatomen, und das enthält, bezogen auf Gewicht, von 96% bis 99,9% Ethylenoxid und von 4% bis 0,1% des 1,2-Epoxyalkans, wobei die Zusammensetzung auch von 10% bis 95 Gew.-% eines Alkalimetall- oder Calciumsalzes von Carboxymethylcellulose enthält.

5. Trockene Haftzusammensetzung für Zahnprothesen, dadurch gekennzeichnet, daß sie als ihren Hauptbestandteil enthält ein hydrophob modifiziertes wasserlösliches Polymer, ausgewählt aus der Gruppe bestehend aus einem hydrophob modifizierten Celluloseether, substituiert mit einem Kohlenwasserstoffrest mit 8 bis 25 Kohlenstoffatomen in einer Menge von 0,2 Gew.-% bis zu der Menge, die den Celluloseether weniger als 1 Gew.-% löslich in Wasser macht, und einem Copolymer von Ethylenoxid und mindestens einem langkettigen 1,2-Epoxyalkan mit 8 bis 25 Kohlenstoffatomen, und das enthält, bezogen auf Gewicht, von 96% bis 99,9% Ethylenoxid und von 4 bis 0,1% des 1,2-Epoxyalkans, wobei die Zusammensetzung auch 0,1% bis 60 Gew.-% eines Füllmaterials enthält.

6. Zusammensetzung nach Anspruch 5, dadurch gekennzeichnet, daß das Füllmaterial Dicalciumphosphat, Calciumcarbonat oder Talk ist.

7. Zusammensetzung nach Anspruch 5 oder 6, dadurch gekennzeichnet, daß sie auch von 10% bis 95 Gew.-% eines Alkalimetall- oder Calciumsalzes von Carboxymethylcellulose enthält.

8. Zusammensetzung nach Anspruch 4 oder 7, dadurch gekennzeichnet, daß das Salz der Carboxymethylcellulose einen D.S. von mindestens 0,3 hat.

9. Zusammensetzung nach Anspruch 2, 3, 4, 7 oder 8, dadurch gekennzeichnet, daß das Salz von Carboxymethylcellulose ein Natriumsalz ist.

10. Zusammensetzung nach einem der Ansprüche 1 bis 9, dadurch gekennzeichnet, daß sie nur den hydrophob modifizierten Celluloseether als das wasserlösliche Polymer enthält.

11. Zusammensetzung nach einem der Ansprüche 1 bis 10, dadurch gekennzeichnet, daß der Celluloseether, der hydrophob modifiziert ist, eine Alkyl- oder Hydroxyalkylcellulose ist.

12. Zusammensetzung nach Anspruch 11, dadurch gekennzeichnet, daß der Celluloseether, der hydrophob modifiziert ist, Hydroxyethylcellulose ist.

13. Zusammensetzung nach einem der Ansprüche 1 bis 9, dadurch gekennzeichnet, daß sie nur das Copolymer von Ethylenoxid und 1,2-Epoxyalkan als das wasserlösliche Polymer enthält.

14. Zusammensetzung nach einem der Ansprüche 1 bis 13, dadurch gekennzeichnet, daß der Kohlenwasserstoffrest oder 1,2-Epoxyalkan 12 bis 20 Kohlenstoffatome hat.

**Revendications**

1. Composition adhésive pour dentier, contenant de 35% à 85% en poids d'un pétrolatum et de 0% à 15% en poids d'une matière choisie dans le groupe constitué par une huile minérale et une huile minérale mélangeze avec un polyéthylène, caractérisée en c qu'elle contient aussi de 5% à 50% en poids d'un polymère hydrosoluble modifié dans le sens de l'hydrophobie, choisi dans le groupe constitué par un éther cellulosique modifié dans le sens de l'hydrophobie, substitué par un radical hydrocarboné à 8—25 atomes de carbone dans une proportion allant de 0,2% en poids à environ la quantité qui rend l'éther cellulosique soluble dans l'eau à moins de 1% en poids, et par un copolymère d'oxyde d'éthylène et d'au moins un 1,2-époxyalcane à longue chaîne comportant de 8 à 25 atomes de carbone, copolymère qui contient, en poids, de 96% à 99,9% d'oxyde d'éthylène et de 4% à 0,1% du 1,2-époxyalcane.

2. Composition selon la revendication 1, contenant de 40% à 80% en poids du pétrolatum, de 0% à 10% en poids de la matière choisie dans le groupe constitué par une huile minérale et une huile minérale mélangée avec un polyéthylène, et de 5% à 35% en poids du polymère hydrosoluble modifié dans le sens de l'hydrophobie, caractérisée en ce qu'elle contient aussi de 10% à 45% en poids d'un sel de métal alcalin ou de calcium et de carboxyméthylcellulose.

3. Composition selon la revendication 2, caractérisée en ce que ledit sel de carboxyméthylcellulose a un degré de substitution (D.S.) d'au moins 0,3.

4. Composition adhésive sèche pour dentier, caractérisée en ce qu'elle contient, en tant qu'ingrédient principal, un polymère hydrosoluble mod fié dans le sens de l'hydrophobie, choisi dans le groupe constitué par un éther cellulosique modifié dans le sens de l'hydrophobie, substitué par un radical hydrocarboné à 8—25 atomes de carbone dans une proportion allant de 0,2% en poids à environ la quantité qui rend l'éther cellulosique soluble dans l'eau à moins de 1% en poids, et par un copolymère d'oxyde d'éthylène et d'au moins un 1,2-époxyalcane à longue chaîne comportant de 8 à 25 atomes de carbone, copolymère qui contient, en poids, de 96% à 99,9% d'oxyde d'éthylène et de 4% à 0,1% du 1,2-époxyalcane, la composition contenant également de 10% à 95% en poids d'un sel de métal alcalin ou de calcium et de carboxyméthylcellulose.

6

5. Composition adhésive sèche pour dentier, caractérisée en ce qu'elle contient, en tant qu'ingrédient principal, un polymère hydrosoluble modifié dans le sens de l'hydrophobie, choisi dans le groupe constitué par un éther cellulosique modifié dans le sens de l'hydrophobie, substitué par un radical hydrocarboné à 8—25 atomes de carbone dans une proportion allant de 0,2% en poids à environ la quantité qui rend l'éther cellulosique soluble dans l'eau à moins de 1% en poids, et par un copolymère d'oxyde d'éthylène et d'au moins un 1,2-époxyalcane à longue chaîne comportant de 8 à 25 atomes de carbone, copolymère qui contient, en poids, de 96% à 99,9% d'oxyde d'éthylène et de 4% à 0,1% du 1,2-époxyalcane, la composition contenant également de 0,1% à 60% en poids d'une charge.

6. Composition selon la revendication 5, caractérisée en ce que la charge est le phosphate dicalcique, le carbonate de calcium ou le talc.

7. Composition selon la revendication 5 ou 6, caractérisée en ce qu'elle contient également de 10% à 95% en poids d'un sel de métal alcalin ou de calcium et de carboxyméthylcellulose.

8. Composition selon la revendication 4 ou 7, caractérisée en ce que ledit sel de carboxyméthylcellulose a un degré de substitution (D.S.) d'au moins 0,3.

9. Composition selon l'une quelconque des revendications 2, 3, 4, 7 ou 8, caractérisée en ce que le sel de carboxyméthylcellulose est un sel sodique.

10. Composition selon l'une quelconque des revendications 1 à 9, caractérisée en ce qu'elle ne contient que l'éther cellulosique modifié dans le sens de l'hydrophobie en tant que ledit polymère hydrosoluble.

11. Composition selon l'une quelconque des revendications 1 à 10, caractérisée en ce que l'éther cellulosique qui est modifié dans le sens de l'hydrophobie est une alkyl- ou hydroxyalkylcellulose.

12. Composition selon la revendication 11, caractérisée en ce que l'éther cellulosique qui est modifié dans le sens de l'hydrophobie est l'hydroxyéthylcellulose.

13. Composition selon l'une quelconque des revendications 1 à 9, caractérisée en ce qu'elle ne contient que ledit copolymère d'oxyde d'éthylène et de 1,2-époxyalcane en tant que ledit polymère hydrosoluble.

14. Composition selon l'une quelconque des revendications 1 à 13, caractérisée en ce que ledit radical hydrocarboné ou ledit 1,2-époxyalcane comporte de 12 à 20 atomes de carbone.